(19) Europäisches Patentamt
European Patent Office
Office européen des brevets

(11) EP 3 495 393 A1

(12) **EUROPEAN PATENT APPLICATION**

(43) Date of publication:
**12.06.2019 Bulletin 2019/24**

(21) Application number: **17382840.1**

(22) Date of filing: **11.12.2017**

(51) Int Cl.:
***C07K 17/04*** (2006.01) ***C09K 11/06*** (2006.01)
***H05B 33/14*** (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(71) Applicant: **Fundación Imdea Materiales**
**28938 Getafe - Madrid (ES)**

(72) Inventors:
- **COSTA RIQUELME, Rubén Darío**
**E-28938 Getafe, Madrid (ES)**
- **FERNÁNDEZ AGUINO, Carmen**
**E-28938 Getafe, Madrid (ES)**

(74) Representative: **ABG Intellectual Property, S.L.**
**Avenida de Burgos, 16D**
**Edificio Euromor**
**28036 Madrid (ES)**

(54) **LONG-LIVING BIO-HYBRID LIGHT-EMITTING DIODE**

(57) The present invention refers to a rigid material composition containing a luminescent protein immobilized therein, a process for preparing it, as well as to a hybrid light emitting-diode comprising a light-emitting diode and a color down-converting encapsulation material, said color down-converting encapsulation material comprise at least one layer of said rigid material composition containing a luminescent protein immobilized therein.

EP 3 495 393 A1

## Description

### FIELD OF THE INVENTION

The present invention relates to the field of light-emitting diodes (also known as LEDs) and more particularly to LEDs based on hybrid materials containing luminescent proteins immobilized in rigid polymer matrices.

### BACKGROUND

White light-emitting diodes (WLEDs) are considered a key solid-state lighting source to replace energy inefficient incandescent light bulbs and environmentally not friendly fluorescent lamps in the near future. They relays on inorganic-based color down-converting materials, most frequently phosphors of rare-earth elements (Park JK et al., Appl Phys Lett. 2004, 84, 1647; Jang HS et al., Appl Phys Lett. 2007, 90, 041906; Xie R-J et al., Nitride Phosphors and Solid-State Lighting, CRC Press, 2011; Tolhurst TM et al., Adv Opt Mater. 2015, 3, 546; Zhang R et al., Laser Photon Rev. 2014, 8, 158). As shown in Figure 1, WLEDs consist of four main components, namely i) the electronic driver, ii) the heat dissipating system, the emitting chip mounted onto the heat sink, iii) a packaging (encapsulation and protection layer) for environmental protection and optical purposes - *i.e.,* light extraction and color conversion, and iv) the color down-converting compound either placed direct onto the emitting chip or dispersed in the encapsulation part.

Although these devices show excellent device stabilities, there are several limitations:

i) *Color quality*: The most efficient WLEDs relay on a blue-emitting GaInN-chip covered by a color down-converting inorganic phosphor based on yellowish orange-emitting rare-earth doped yttrium aluminum garnets, showing poor color quality with color rendering index (CRI) values *ca.* 75-80 - *i.e.,* bluish-white or cold white.(J. Am. Ceram. Soc., 2014, 97, 1327-1352; J. Sol. State Light 2014 1, 11). This has both visual and non-visual effects on the human health like, for example, the disruption of the circadian rhythm (Environ. Health Perspect. 2014, 122, 269; Curr. Pharm. Des. 2015, 21, 3428; Neuroscience 2016, 339, 296; *In* Innovative Materials: Engineering and Applications II; Key Engineering Materials; Trans Tech Publications, 2017, 730, 112]. However, radiation intensities *of ca.* 100 $kW/m^2$ of blue-chips have blocked the use of organic color down-converting phosphors up to date.

ii) *Costs and sustainability of LED components.* WLEDs still represent less than 0.1 % of the worldwide lighting waste stream (Lighting Research & Technology 2017 https://doi.org/10.1177/1477153517708597). In fact, they are based on rare-earth and/or nobel elements that are scarce and its production is associated to an ecological impact regarding mining, transporting, refining at high temperatures, *etc.* Two major concerns are the cost and the potentially limited supply. As such, the EU Sustainable Development Strategy Commission strongly encourages their further advance using eco-friendly and highly stable optical systems and active materials, as well as the establishment of efficient and low-cost recycling protocols for LEDs.

These limitations has led to a new research field called hybrid inorganic/organic LEDs, in which the inorganic phosphors are replaced for new organic-based color down-converting coating based on sustainable and non-toxic compounds, such as luminescent polymers, carbon quantum dots, carbon nanodots, small molecules, coordination complexes, and, more recently fluorescent proteins (Heliotis G et al., Adv Mater. 2006, 18, 334; Gu E. et al., Appl Phys Lett. 2007, 90, 031116; Huyal IO et al., J Mater Chem. 2008, 18, 3568; Kim O et al., ACS Nano 2010, 4, 3397; Stupca M et al., J Appl Phys. 2012, 112, 074313; Ban D et al., Phys Status Solidi Curr Top Solid State Phys. 2012, 9, 2594; Jang E-P et al., Nanotechnology 2013, 24, 045607; Findlay NJ et al., J Mater Chem C 2013, 1, 2249; Lai C-F et al., Opt Lett. 2013, 38, 4082; Chen J et al., J Mater Sci. 2014, 49, 7391; Kim J-Y, Opt Commun. 2014, 321, 86; Shen P-C et al., Sci Rep. 2014, 4, 5307; Findlay NJ et al., Adv Mater. 2014, 26, 7290).

Common to all these devices is the use of a similar LED architecture - *i.e.,* high-energy emitting inorganic LED with maxima emission wavelength ranging from 360 to 470 nm-, in which the current packaging system is replaced by a new coating based on a matrix and the organic color down-converting. Typical matrix components involve: i) thin films with a mixture of organic materials with UV- or thermal-curable sealing reagents (Adv. Mater., 2006, 18, 334; J. Mater. Chem., 2008, 18, 3568; ACS Nano, 2010, 4, 3397; Nanotechnology, 2013, 24, 045607; Sci. Rep., 2014, 4, 5307; Adv. Mater., 2014, 26, 7290; Nanoscale, 2015, 7, 12045). They are typically deposited either onto a glass substrate or on top of the packing of the LED. Other typical matrix includes metal-organic frameworks (Nat. Commun., 2013, 4, 2717; Adv. Funct. Mater., 2015, 25, 4796), cellulose/silk/mucin derivatives (J. Mater. Chem. C, 2015, 3, 3536; ACS Appl. Mater. Interfaces, 2015, 7, 15830.), and an elastomeric coating based on physical cross-linked polymers to embed all kind of luminescent compounds (Mater. Horiz., 2016, 3, 340) including fluorescent proteins (Adv. Mater., 2015, 27, 5493; Adv. Funct. Mater., 2017, 27, 1601792). This architecture has recently led to hybrid LEDs with high colour quality - *i.e.*, commission international de l’E’ clairage (CIE) coordinates of 0.30-3/0.30-3, colour rendering index (CRI) above 90, and

correlated colour temperature (CCT) between 2500-6500 K, but still with low stabilities of around 100 h due to degradation of the luminescent material, the matrix, or both upon continuous excitation under ambient conditions.

Within the field of hybrid LEDs, particular mention should be made to those based on fluorescent proteins as colour down-converting components, hereafter called Bio-LEDs.

The Bio-LED technology based on the use of said fluorescent proteins is restricted to a handful number of contributions which are herein below described.

Hui, K.N. (Nanotechnology, 2008, 19, 355203) discloses Bio-LEDs based on fluorescent proteins entrapped into polystyrene microspheres and deposited onto the LED emitting chip. A further protective layer - *i.e.,* epoxy - is deposited onto the thin protein-based thin film. As such, this procedure consists of two fabrication steps, being the polystyrene microspheres with a fluorescent protein core a commercial hybrid compound needed due to the incompatibility between said fluorescent proteins and the final protective epoxy layer. However, the matrix stability is not described and, in fact, the presence of an epoxy protective layer is required.

WO2017/039406 also describes a biomaterial comprising fluorescent proteins and a protective layer made of an epoxy resin. Since, as mentioned before, proteins are incompatible with an epoxy layer as they cannot endure in such a polymer resin, they are mixed with egg white leading to a hybrid protein-$DNA_{egg}$ in which the fluorescent proteins are cross-linked to the DNA present in the egg white. Furthermore, the addition of a surfactant is needed to produce a precipitate that is collected and further dispersed in the protective layer, although it is unclear the effect of said surfactant as stabilizer to allow the use of commercial epoxy. Anyway, there is no description of a polymer matrix to stabilize the fluorescent proteins (not only to protect them) or to a rigid coating lacking the use of standard protective layers of epoxy compounds. Furthermore, this type of Bio-LEDs does not circumvent the problems mentioned above. In addition to that, the device stability, efficiency and color quality are not disclosed.

Bio-LEDs based on a dried film of fluorescent proteins without using any matrix component and/or a further encapsulation is described in Fen Bilim. Enstitüsü Derg., 2016, 20, 490; and Nanotechnology 2016, 27, 45LT01. The device stability is not disclosed, but the authors only commented on the possibility to enhance the device stability using additives like polysaccharides.

WO2016/203028 (see also Adv. Mater., 2015, 27, 5493; Adv. Funct. Mater., 2017, 27, 1601792) describes Bio-LEDs based on fluorescent proteins entrapped into a rubber-like coating based on a mixture of a branched and a linear polyethylene oxide compounds in aqueous solution. This coating replaces the commercial packaging and it does not include a further encapsulation. The procedure and examples disclosed relates to the use of hydrophilic branched and linear polymers that are mixed in aqueous solutions containing the fluorescent proteins. Upon drying under vacuum conditions, the gel-like materials transform into a rubber-like and/or elastomeric material.

Although Bio-LEDs based on a rubber-like and/or elastomeric fluorescent protein packaging show less than 10% loss in luminous intensity over a 100 h upon driving the device at low applied currents (10 mA), they show a low stability of a few minutes (< 5 min) upon driving the device at high applied currents (200 mA). A rationale, which has not been disclosed in the literature yet, is the high temperature reached by the coating upon continuous excitation.

The increase in temperature is detrimental with respect to the intrinsically low thermal stability of fluorescent proteins and the rapid loss of water molecules embedded into the elastomeric matrix. Both aspects are limitations of the rubber-like material in terms of low thermal conductivity and high degree of flexibility that facilitates the loss of water. An obvious solution is the use of a protective layer covering the rubber-coating as described in other examples (Nanotechnology, 2008, 19, 355203). However, this is not desired as it increases the number of fabrication steps and it could potentially be incompatible due to the use of hydrophobic compounds for both matrix and protective layers, being fluorescent proteins only stable enough for lighting applications using aqueous solutions or elastomeric materials containing a high percentage of water molecules.

As such, the main technical challenges in the field of Bio-LEDs based on fluorescent proteins as colour down-converting packaging are:

- need of a new matrix to reduce the thermal stress and loss of water to preserve the device stability without using a further protective layer.
- need of new fabrication procedure not limited to the use of hydrophilic polymers and/or aqueous solutions.

None of the disclosed documents have proposed methods to solve the issues above. As such, there is a strong need of Bio-LEDs having higher stabilities, avoiding the degradation of the fluorescent proteins upon continuous excitation under ambient conditions and at high applied currents, as well as the presence of incompatible coatings.

## BRIEF DESCRIPTION OF THE INVENTION

The authors of the present invention have found a technical solution that simultaneously solves the above-discussed problems of the Bio-LEDs described in the prior art. More particularly, they have developed a single-layer

and rigid polymer coating in which the embedded luminescent proteins are stable over time, as said polymer coating reduces the temperature and loss of water at high applied currents, surprisingly leading to a 10-fold enhancement of the stability. In fact, the temperature reduction not only reduces the degradability of the proteins but also that of polymer-containing rigid coating.

Furthermore, the Bio-LEDs can be prepared in a very simple way by means of a process based on the combination of proteins with hydrophilic/hydrophobic polymer matrix using orthogonal solvents, without affecting the stability of said proteins.

Thus, a first aspect of the present invention refers to a rigid material composition containing a luminescent protein immobilized therein, wherein the material composition further comprises a hydrophilic star-like polymer and a hydrophobic thermoplastic polymer, said hydrophobic thermoplastic polymer having a Tg higher than 30°C, and wherein the hydrophilic star-like polymer comprises at least three polymeric branches bound to a central branching unit.

A second aspect of the invention relates to a process (from now onwards process 1) for producing a material composition as defined above, said process comprising:

a) mixing the luminescent protein and the star-like polymer in an aqueous solution;

b) adding to the mixture resulting from step a) a solution of a hydrophobic thermoplastic polymer having a Tg higher than 30°C in an orthogonal solvent to water, to obtain a gel-like material; and

c) drying the gel obtained in step b) to obtain a rigid material composition containing the luminescent protein immobilized therein.

A third aspect of the invention relates to a rigid material composition obtainable by the process as defined above.

A fourth aspect of the invention refers to a gel-like material comprising a luminescent protein, a hydrophilic star-like polymer and a hydrophobic thermoplastic polymer, said hydrophobic thermoplastic polymer having a Tg higher than 30°C, and wherein the hydrophilic star-like polymer comprises at least three polymeric branches bound to a central branching unit.

The invention also relates to a process (from now onwards process 2) for preparing a gel-like material as defined above, said process comprising:

a) mixing the luminescent protein and the star-like polymer in an aqueous solution; and

b) adding to the mixture resulting from step a) a solution of a hydrophobic thermoplastic polymer having a Tg higher than 30°C in a solvent which is orthogonal to water.

Another aspect of the invention relates to the use of the rigid material composition in lightning, particularly as an environmentally friendly colour down-converting encapsulation material for a hybrid light-emitting diode.

Accordingly, another aspect of the invention refers to a hybrid light-emitting diode comprising a light-emitting diode and a colour down-converting encapsulation material, said colour down-converting encapsulation material comprising at least one layer of rigid material composition containing a luminescent protein immobilized therein as defined in the first or third aspect of the invention. The luminescent protein contained in the material composition is preferably a fluorescent protein.

## BRIED DESCRIPTION OF THE FIGURES

Figure 1 shows a sketch of a Bio-hybrid light-emitting diode (Bio-LED) according to the invention

Figure 2 shows the device performance of a Bio-LED with a rubber-like coating, showing the normalized decay of the emission intensity (dotted line) of the fluorescent protein and the rise of the temperature (solid line) of the coating upon driving at a current of 200 mA.

Figure 3 shows the device performance of a Bio-LED with a rigid coating, showing the normalized decay of the emission intensity (dotted line) of the fluorescent protein and the rise of the temperature (solid line) of the coating upon driving at a current of 200 mA

## DETAILED DESCRIPTION OF THE INVENTION

As mentioned above, the first aspect of the invention relates to a rigid material composition containing a luminescent protein immobilized therein, wherein the material composition comprises a hydrophilic star-like polymer and a hydrophobic rigid thermoplastic polymer, said hydrophobic rigid thermoplastic polymer having a Tg higher than 30°C, and wherein the hydrophilic star-like polymer comprises at least three polymeric branches bound to a central branching unit.

In the context of the present invention, the term "rigid material composition" refers to a material composition that is essentially non-flexile under conventional forces exerted by a person.

The presence of the hydrophobic thermoplastic polymer having a Tg higher than 30°C provides a rigid material composition wherein the luminescent protein is immobilized. Said rigidity has found to impair a high thermal stability to the Bio-LED where this rigid material composition is incorporated as an encapsulation, as no temperature increase is observed, thus reducing significantly the degradability of luminescent proteins. Furthermore, the loss of water is also remarkably reduced, not only due to the rigidity of the resulting polymeric matrix, but also because the process to obtain the material composition requires less water addition. In fact, and as shown in the experimental results, a 10-fold enhancement in the device stability has been surprisingly found.

As mentioned above, the hydrophobic thermoplastic polymer comprised in the material composition of the invention is characterized for having a Tg higher than 30°C, preferably higher than 40°C, more preferably higher than 50°C, even more preferably higher than 70°C. In other words, this thermoplastic polymer is one which at ambient temperature is solid and rigid. It is this component of the material composition which is primarily responsible for the hardness and rigidity characteristics thereof.

In the context of the present invention, the values of "glass transition temperature" (Tg) refer to those taken from the second heating in differential scanning calorimeter (DSC) experiments. Said experiments can be carried out under non-isothermal (10°C/min from -85 to 200°C) conditions using a DSC TA Instruments Q2000, operating with an intra-cooler under nitrogen flow. Temperature and heat flow calibrations are performed with indium as standard.

Also in the context of the invention, the "number average molecular weight" (or "Mn") of a polymeric component refers to the average (arithmetic mean) of the molecular weights of the individual molecules of the corresponding component (e.g., of the branched polymer or of the linear polymer). The number average molecular weight (Mn) is defined as follows:

$$Mn = \frac{\sum_i NiMi}{\sum_i Ni}$$

wherein Ni is the number of molecules of the respective component having a molecular weight Mi, and the summation includes all molecular weights of the corresponding component that are present. The number average molecular weight of a component (such as the branched polymer) can be determined using methods known in the art, such as e.g., by gel permeation chromatography (GPC), viscosity measurements (viscometry), osmotic-pressure measurements, light-scattering measurements (e.g., using the Zimm method), colligative methods (such as vapor-pressure osmometry, boiling-point elevation, freezing-point depression, or vapor-pressure lowering), endgroup determination, or 1H-NMR.

It is preferred that the number average molecular weight is to be determined using GPC or viscosity measurements, more preferably by GPC. The GPC system can be calibrated, e.g., relative to a set of anionically polymerized polystyrenes having a dispersity Mw/Mn < 1.10 (ideally Mw/Mn = 1) as calibration standard. For example, the number average molecular weight can be determinad by GPC, using a GPC apparatus C0-8011 (Tosoh Bioscience LLC) equipped with a column GMHHR-H (Tosoh Bioscience LLC), using tetrahydrofuran as the solvent, measuring at 40°C, and using polystyrenes as standard (e.g., as described above).

Water can also be used as the solvent instead of tetrahydrofuran in this method. Alternatively, the number average molecular weight can be determined by GPC, using a GPC apparatus 150C (Waters Corporation) equipped with a Shodex Packed Column A-80M (Showa Denko K.K.), measuring at 140°C, using ortho-dichlorobenzene as solvent/carrier, a flow rate of 1.0 mL/min, a sample concentration of about 1 mg/mL, an injection amount of 400 ml, a differential refraction detector, and polystyrenes as standard (e.g., as described above). It is particularly preferred that the number average molecular weight is determined by GPC, using a GPC apparatus 150C (Waters Corporation) equipped with a Shodex Packed Column A-80M (Showa Denko K.K.), measuring at 40°C, using water as solvent, a flow rate of 1.0 mL/min, a sample concentration of about 1 mg/mL, an injection amount of 400 ml, a differential refraction detector, and polystyrenes as standard (e.g., anionically polymerized polystyrenes having a dispersity Mw/Mn < 1.10).

In a particular embodiment, the hydrophobic thermoplastic polymer exhibits a Shore "D" hardness of 40 or higher according to ASTM Standard D2240 and/or exhibits a flex modulus above 15.000 psi according to ASTM Standard D790.

The hydrophobic thermoplastic polymer has preferably a transparency degree of at least 90% in the region of the visible spectra in order to ensure a suitable transparency.

Thus, in a preferred embodiment, the hydrophobic thermoplastic polymer has an amorphous structure or has a crystallinity degree of not more than 80%. More preferably, it has an amorphous structure.

In a particular embodiment of the invention, said hydrophobic thermoplastic polymer is selected from poly (meth)acrylates, polystyrenes, polycarbonates, polyesters, vinyl halide polymers, polyacrylamides, polypropylene, polybenzymidazoles, polyethersulfones, polyetheretherketones, polyetherimides, polyphenylene oxides, polyphenylene sulphides, polyamides, polytetrafluoroethylenes, and mixtures thereof, provided that they have a Tg higher than 30°C and a degree of transparency in the visible spectra higher than 90%.

Said hydrophobic thermoplastic polymers suitable of use in the invention are commercially available or can be prepared according to standard procedures known in the prior art, such as, mass polymerization, emulsion polymerization, suspension polymerization or combinations thereof.

In a preferred embodiment, the hydrophobic thermoplastic polymer is selected from poly (meth)acrylates, vinyl halide polymers, polystyrenes, polycarbonates, and mixtures thereof.

In a preferred embodiment, the hydrophobic thermoplastic polymer is a poly (meth)acrylate. The term poly(meth)acrylates refers to those polymers having repeating units derived from one or more monoethylenically unsaturated carboxylic acids or esters thereof, for example, acrylic acid; methacrylic acid; itaconic acid; ($C_1$-$C_{12}$)alkyl (meth)acrylate monomers, such as methyl (meth)acrylate, ethyl(meth)acrylate; ($C_4$-$C_{12}$)cycloalkyl(meth)acrylate monomers, such as cyclohexyl(meth)acrylate. In a more preferred embodiment, the rigid hydrophobic thermoplastic polymer is a polymer having repeating units derived from ($C_1$-$C_{12}$)alkyl (meth)acrylate monomers, more preferably from ($C_1$-$C_4$)alkyl (meth)acrylate monomers, even more preferably from methyl methacrylate monomers.

In another preferred embodiment, the hydrophobic thermoplastic polymer is a polystyrene. The term "polystyrene" refers to those polymers having repeating units derived from vinyl aromatic monomers, such as, for example, styrene and substituted styrenes having one or more alkyl, alkoxy, hydroxy or halo substituent groups attached to the aromatic ring, including, but not limited to, alpha-methyl styrene, p-methyl styrene, 3,5-diethylstyrene, 4-n-propylstyrene, 4-isopropylstyrene, vinyl toluene, alpha-methyl vinyl toluene, vinyl xylene, trimethyl styrene, butyl styrene, t-butyl styrene, chlorostyrene, alpha-chlorostyrene, dichlorostyrene, tetrachlorostyrene, bromostyrene, alpha-bromostyrene, dibromostyrene, p-hydroxystyrene, p-acetoxystyrene, methoxystyrene and vinyl-substituted condensed aromatic ring structures, such as, for example, vinyl naphthalene, vinyl anthracene. Substituted styrenes with mixtures of substituents on the aromatic ring are also suitable. In a more preferred embodiment, the rigid hydrophobic thermoplastic polymer is a polymer having repeating units derived from styrene.

Homopolymers are preferred, although copolymers (both random and block copolymers) of any of the aforementioned polymeric species are possible.

In a preferred embodiment, the hydrophobic thermoplastic polymer is selected from homopolymers of (meth)acrylates, such as poly(methyl methacrylate); vinyl halide polymers, such as polyvinyl chloride; polystyrenes; polycarbonates. Even more preferably, it is a poly(methyl methacrylate) or a polystyrene. Even much more preferably, the rigid hydrophobic thermoplastic polymer is poly(methyl methacrylate).

In a particular embodiment, the hydrophobic thermoplastic polymer is a copolymer comprising structural units derived from at least one vinyl aromatic monomer, such as those mentioned above, and at least one monoethylenically unsaturated nitrile monomer.

The term "monoethylenically unsaturated nitrile monomer" means an acyclic compound that includes a single nitrile group and a single site of ethylenic unsaturation per molecule and includes, but is not limited to, acrylonitrile, methacrylonitrile, ethacrylonitrile, alpha-chloroacrylonitrile, alpha-bromoacrylonitrile, and the like.

Examples of such copolymers include, but are not limited to, styrene/acrylonitrile copolymers, alpha-methylstyrene/acrylonitrile copolymers, or alphamethylstyrene/styrene/acrylonitrile copolymers.

In another particular embodiment, the hydrophobic thermoplastic polymer is a copolymer comprising structural units derived from at least one vinyl aromatic monomer; from at least one monoethylenically unsaturated nitrile monomer; and from at least one monomer selected from the group consisting of ($C_1$-$C_{12}$)alkyl- and aryl-(meth)acrylate monomers, such as those mentioned above. Examples of such copolymers include, but are not limited to, styrene/acrylonitrile/methyl methacrylate copolymers, alpha-methylstyrene/acrylonitrile/methyl methacrylate copolymers and alpha-methylstyrene/styrene/acrylonitrile/methyl methacrylate copolymers. These copolymers may be used for the rigid hydrophobic thermoplastic polymer either individually or as mixtures.

Selection of the optimum hydrophobic thermoplastic polymer may depend upon the physical properties required for the material composition, in particular of the rigidity of said material composition, which may depend at least to some extend on the amount of it to be incorporated in the composition.

Thus, in a particular embodiment, the rigid material composition of the invention preferably comprises the hydrophobic thermoplastic polymer in an amount within the range from 20 to 80 wt%, more preferably from 30 to 70 wt%, even more preferably from 40 to 60 wt%, even much more preferably from 45 to 55 wt%, based on the total weight

of the material composition.

In another particular embodiment, the number average molecular weight (Mn) of the hydrophobic thermoplastic polymer ranges from $10^3$ to $10^7$ Da, more preferably from $10^4$ to $10^6$ Da, even more preferably from $10^4$ to $10^5$ Da. In this sense, the higher the number average molecular weight of the hydrophobic thermoplastic polymer, the lower its weight proportion in the material composition of the invention with respect to the hydrophilic star-like polymer. Thus, the number average molecular weight and weight proportion of the hydrophobic thermoplastic polymer should be such that the resulting material composition is rigid at ambient temperature.

In a preferred embodiment of the invention, the hydrophobic thermoplastic polymer is poly(methyl methacrylate) having a Mn ranging from 100.000 to 200.000 Da. More preferably, the material composition of the invention preferably comprises said poly(methyl methacrylate) in an amount ranging from 45 to 55 wt% based on the total weight of the material composition.

The other polymeric component of the material composition of the invention is a star-like polymer.

By the term "star-like polymer" should be understood a polymer comprising at least one moiety or central core to which at least three polymer chains or branches are covalently bonded. Each branch can be chemically identical or different.

Thus, the star-like polymer to be used in accordance with the present invention comprises at least three polymeric branches covalently bound to a central core. Furthermore, said star-like polymer is hydrophilic, understanding as such a polymer having functional groups capable of forming hydrogen bonding, thus being able to be absorbed or dissolved in water.

The central core comprised in the star-like polymer is preferably a $C_{1-20}$ hydrocarbon moiety which is substituted with 3 to 8 substituent groups, wherein said substituents groups are each independently selected from hydroxyl, carboxyl and amino, and further wherein each of the at least three polymeric branches of the star-like polymer is covalently bound to one of the substituent groups of the $C_{1-20}$ hydrocarbon moiety. Optionally, one or more carbon atoms (e.g., one, two, three, four or five carbon atoms) of the $C_{1-20}$ hydrocarbon moiety are each independently replaced by an oxygen atom, a nitrogen atom or a sulfur atom, preferably by an oxygen atom. It is to be understood that each one of the at least three polymeric branches is bound to a different substituent group on the above-mentioned hydrocarbon moiety, and that the maximum number of polymeric branches that can be bound to the central core is the same as the maximum number of substituent groups on the hydrocarbon moiety.

The above-mentioned hydrocarbon moiety is preferably a $C_{3-20}$ hydrocarbon moiety, more preferably a $C_{3-15}$ hydrocarbon moiety and even more preferably to a $C_{4-10}$ hydrocarbon moiety. The hydrocarbon moiety is preferably substituted with 3, 4, 5 or 6 substituents groups, more preferably with 3, 4 or 5 substituent groups, and even more preferably with 3 or 4 substituent group, and yet even more preferably with 3 substituent groups.

The substituent groups on the hydrocarbon moiety are each independently selected from hydroxyl (-OH), carboxyl (-COOH) and amino ($-NH_2$), and are preferably each hydroxyl. If the substituent group which is attached to a polymeric branch is a hydroxyl group, then it is preferred that the polymeric branch is attached to said hydroxyl group via an ether linkage or via an ester linkage, more preferably via an ether linkage. If the substituent group which is attached to a polymeric branch is a carboxyl group, then it is preferred that the polymeric branch is attached to said carboxyl group via an ester linkage or via an amide linkage. If the substituent group which is attached to a polymeric branch is an amine group, then it is preferred that the polymeric branch is attached to said amine group via an amide linkage.

Accordingly, it is preferred that the central core is a $C_{3-20}$ hydrocarbon moiety which is substituted with 3 to 8 substituent groups, wherein said substituent groups are each independently selected from hydroxy, carboxy and amino, optionally wherein one or more carbon atoms (e.g., one, two, three, four or five carbon atoms) comprised in said $C_{3-20}$ hydrocarbon moiety are each replaced by an oxygen atom, and further wherein each of the at least three polymeric branches is bound to one of the substituent groups of the $C_{3-20}$ hydrocarbon moiety. More preferably, the central core is a $C_{3-20}$ hydrocarbon moiety which is substituted with 3 to 8 hydroxy groups, optionally wherein one or more carbon atoms (e.g., one, two, three, four or five carbon atoms) comprised in said $C_{3-20}$ hydrocarbon moiety are each replaced by an oxygen atom, and further wherein each of the at least three polymeric branches is bound to one of the hydroxy groups of the $C_{3-20}$ hydrocarbon moiety. Even more preferably, the central core is selected from a trimethylolpropane moiety, a trimethylolethane moiety, a trimethylolmethane moiety, a glycerol moiety, a pentaerythritolmoiety, a pentaerythrithiol moiety, a diglycerol moiety, a triglycerol moiety, a dipentaerythritol moiety, a tetraglycerol moiety, a pentaglycerol moiety, a tripentaerythritol moiety, a hexaglycerol moiety, a trimethanolamine moiety, a triethanolamine moiety, a triisopropanolamine moiety, a propane-1 ,2,3-tricarboxylic acid moiety, a citric acid moiety, an isocitric acid moiety, a trimesic acid moiety, a 1,1,1-tris(aminomethyl)propane moiety, a 1,1,1-tris(aminomethyl)ethane moiety, a tris(aminomethyl)methane moiety, a propane-1,2,3-triamine moiety, a tris(2-aminoethyl)amine moiety, and a tris(carboxymethyl)ethylenediamine moiety. The central core may also be a tri($C_{1-8}$ alkanol)amine moiety or a tri(hydroxy-$C_{1-8}$ alkylene)amine moiety. Still more preferably, the central core is selected from a trimethylolpropane moiety, a trimethylolethane moiety, a trimethylolmethane moiety, and a glycerol moiety. Most preferably, the central core is a trimethylolpropane moiety, as shown in the following formula:

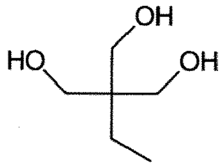

Trimethylolpropane

The at least three polymeric branches comprised in the star-like polymer can all be the same or can be different from one another, and are preferably the same. It is preferred that the star-like polymer has 3 to 8 polymeric branches, more preferably 3, 4, 5 or 6 polymeric branches, even more preferably 3, 4 or 5 polymeric branches, yet even more preferably 3 or 4 polymeric branches, and most preferably 3 polymeric branches. The polymeric branches may be linear or branched, and may also be dendritically branched.

Preferably, each of the polymeric branches is a linear polymer. More preferably, the polymeric branches are each independently a poly(alkylene oxide) having a terminal - OH, -OR, -O-CO-R, -CO-O-R, or-CO-N(R)-R group, wherein each R is independently $C_{1-5}$ alkyl (e.g., methyl or ethyl) or $C_{2-5}$ alkenyl (e.g., vinyl). The poly(alkylene oxide) may be, e.g., a poly(ethylene oxide}, a poly(propylene oxide), or a copolymer of ethylene oxide and propylene oxide (e.g., a block copolymer of ethylene oxide (EO) and propylene oxide (PO), such as a block copolymer having the structure EO-PO-EO), and is preferably a poly(ethylene oxide). Moreover, the poly(alkylene oxide) preferably has a terminal -OH, -OR or -O-CO-R group, more preferably a terminal -OH group. The aforementioned terminal group is present at a different or opposite end of the poly(alkylene oxide) in relation to the point of attachment of the poly(alkylene oxide) to the central core. The polymeric branches comprised in the star-like polymer are even more preferably each independently a poly(ethylene oxide), a poly(propylene oxide), or a copolymer of ethylene oxide and propylene oxide, wherein said poly(ethylene oxide), said poly(propylene oxide) and said copolymer each has a terminal -OH, -OR or -O-CO-R group, wherein each R is independently $C_{1-5}$ alkyl. Yet even more preferably, the polymeric branches are each independently a poly(ethylene oxide) having a terminal - OH group.

It is preferred that the star-like polymer has a number average molecular weight of about 200 Da to about 2000 Da, more preferably of about 300 Da to about 1200 Da. It is furthermore preferred that the star-like polymer is water-soluble at a temperature of 25°C and an absolute pressure of 1 bar (100 kPa).

It is particularly preferred that the star-like polymer is trimethylolpropane ethoxylate (TMPE), the structure of which is illustrated in the following (wherein each variable n denotes the number of repeating ethylene oxide monomer units of the corresponding polymeric branch):

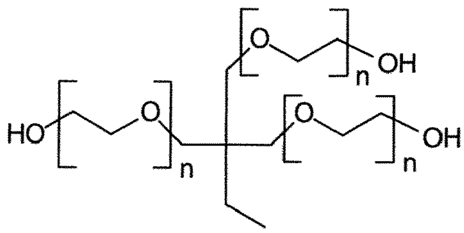

Trimethylolpropane ethoxylate (TMPE)

Even more preferably, the star-like polymer is trimethylolpropane ethoxylate having a number average molecular weight of about 300 Da to about 1200 Da (e.g., about 450 Da, about 730 Da, or about 1040 Da), yet even more preferably the star-like polymer is trimethylolpropane ethoxylate having a number average molecular weight of about 350 Da to about 550 Da, and most preferably the star-like polymer is trimethylolpropane ethoxylate having a number average molecular weight of about 450 Da.

A further example of the star-like polymer is trimethylolpropane ethoxylate methyl etherdiacrylate (TMPEMED), such as TMPEMED having a number average molecular weight of about 70 Da to about 2000 Da, preferably of about 200 Da to about 800 Da, more preferably of about 388 Da:

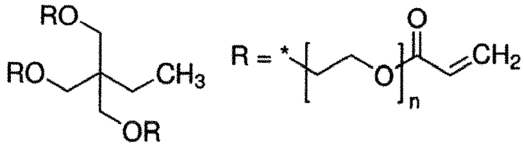

Trimethylolpropane ethoxylate methyl ether diacrylate (TMPEMED)

A further example of the star-like polymer is polyethylenimine (PEI), such as PEI having a weight average molecular weight of about 200 Da to about 3000 Da, preferably of about 400 Da to about 1600 Da, more preferably of about 800 Da:

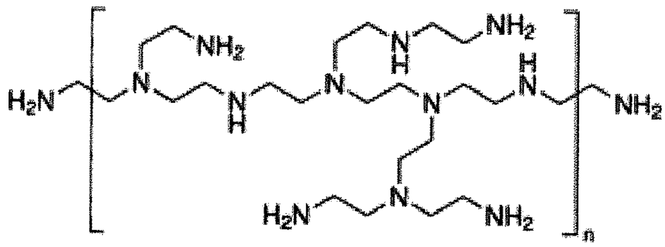

Polyethyleneimine (PEI)

It is possible to use a single star-like polymer, i.e. a single type of star-like polymer, or to use two or more (e.g., two, three, four, or five) different star-like polymers.

In a particular embodiment of the invention, the weight ratio of the hydrophobic thermoplastic polymer to star-like polymer ranges from 1:3 to 3:1, more preferably from 1:2 to 2:1, even more preferably is 1:1.

However, said weight ratio mainly depends on the polymers used, as well as the Tg and the molecular weight of the hydrophobic thermoplastic polymer. As mentioned before, the higher the number average molecular weight of the hydrophobic thermoplastic polymer, the lower its weight proportion in the material composition of the invention with respect to the hydrophilic star-like polymer. In any case, a skilled in the art would know which weight proportion of polymers can be used provided that the resulting material composition is rigid at ambient temperature.

The protein to be used in accordance with the invention is a luminescent protein, more preferably a fluorescent protein.

The term "protein" is used herein interchangeably with "polypeptide" or "peptide" and refers to a polymer of two or more amino acids (preferably 10 or more amino acids, more preferably 20 or more amino acids, even more preferably 50 or more amino acids, even more preferably 100 or more amino acids, even more preferably 150 or more amino acids, and yet even more preferably 200 or more amino acids) linked via amide bonds that are formed between an amino group of one amino acid and a carboxyl group of another amino acid. In the context of the present invention, the protein is luminescent, i.e. spontaneously emits light upon radiative excitation.

The luminescent protein is preferably a fluorescent protein, such as, e.g., green fluorescent protein (GFP), enhanced green fluorescent protein, blue fluorescent protein, cyan fluorescent protein, teal fluorescent protein, yellow fluorescent protein, orange fluorescent protein, red fluorescent protein, near-infrared fluorescent protein, mCherry, mStrawberry, mRaspberry, mOrange, mCitrine, tdTomato, mTagBFP, dsRed, UnaG, eqFP61 1, Dronpa, TagRFPs, KFP, EosFP, Dendra, or IrisFP.

The fluorescent protein may also be a fusion protein comprising a fluorescent protein (e.g., any of the above-mentioned specific luminescent proteins), which is fused, optionally via a linker (e.g., a glycine and/or serine rich linker, such as a linker composed of 2 to 35 amino acids, preferably 5 to 10 amino acids, selected independently from glycine and serine, or any one of the linkers mentioned in Reddy Chichili VP et al., Protein Scí. 2013, 22(2): 153-67, including in Table 1 of this reference), to an adaptor protein domain (e.g., an Src homology 2 domain (SH2 domain), an Src homology 3 domain (SH3 domain), or a poly(A)-binding protein C-terminal domain (PABC domain)). It is possible to use a single protein, i.e. a single type of protein, or to use two or more different proteins, e.g., two or more different luminescent proteins.

In a particular embodiment, the luminescence protein is a conventional protein that has been modified by covalent attachment of one or more non-protein fluorescent dyes (such as fluorescein, rhodamine, Oregon green, eosin, or Texas red) and/or one or more phosphorescent dyes. In this particular case, the protein comprises amino acids, which

are also referred to as amino acid residues, that may be selected from the 20 standard proteinogenic α-amino acids (i.e., Ala, Arg, Asn, Asp, Cys, Glu, Gln, Gly, 25 His, Ile, Leu, Lys, Met, Phe, Pro, Ser, Thr, Trp, Tyr, and Val) but also from non-proteinogenic and/or non-standard α-amino acids (such as, e.g., ornithine, citrulline, homolysine, pyrrolysine, or 4-hydroxyproline) as well as β-amino acids (e.g., 13-alanine), γ-amino acids and δ-aminoacids. Preferably, the amino acid residues comprised in the protein are selected from α-aminoacids, more preferably from the 20 standard proteinogenic α-amino acids (which can be present as the L-isomer or the D-isomer, and are preferably all present as the L-isomer).

It is generally preferred that the luminescent protein is unmodified, unless explicitly indicated otherwise. The amino acid residues comprised in the luminescent protein may, e.g., be present as a linear molecular chain (forming a linear protein) or may form one or more rings (corresponding to a cyclic protein). The protein may also form oligomers consisting of two or more identical or different molecules. The protein may, e.g., comprise or consist of about 200 to about 800 amino acid residues, or it may have a molecular weight of about 20 kDa to about 800 kDa.

The invention also refers to a process (process 1) for preparing the rigid material composition containing a luminescent protein immobilized therein as described above, said process comprising:

a) mixing the luminescent protein and the star-like polymer in an aqueous solution;

b) adding to the mixture resulting from step a) a solution of a hydrophobic thermoplastic polymer having a Tg higher than 30°C in an orthogonal solvent to water, to obtain a gel-like material;

c) drying the gel obtained in step b) to obtain a rigid material composition containing the luminescent protein immobilized therein.

The present invention also relates to a process (process 2) to obtain a gel-like material, said process comprises steps (a) and (b) of the process 1 defined above but not the drying step (c).

The aqueous solution to be used in step (a) of the processes 1 and 2 is not particularly limited, and is preferably water or an aqueous buffer solution. Examples of suitable aqueous buffer solutions include, in particular, phosphate buffer, HEPES buffer, Tris buffer, MOPS buffer, MES buffer, TES buffer, CHES buffer, PIPES buffer, CAPS buffer, HEPPS buffer, imidazole buffer, tricine buffer, bicine buffer, glycine buffer, citric acid buffer, or acetic acid buffer. The pH of the buffer can be adjusted using, e.g., HCl or NaOH (or KOH) as desired for the protein to be immobilized, particularly to a pH at which the protein remains correctly folded (e.g., about pH 6, about pH 7, or about pH 8). A preferred exemplary aqueous buffer solution is phosphate-buffered saline (PBS), which can be prepared, e.g., according to the Cold Spring Harbar Protocol (doi:1 0.11 01/pdb.rec8247). It is particularly preferred that the protein is provided in an aqueous buffer solution containing 50 mM $NaH_2PO_4$ pH 8.0, 300 mM NaCl, and 250 mM imidazole (e.g., at a protein concentration of about 1 mg/ml to about 20 mg/ml). The aqueous buffer solution may further contain one or more protein stabilizers, such as poly(ethyleneimine) (PEI), ethylenediaminetetraacetic acid (EDTA), ammonium sulfate, trehalose, or a commercially available protein stabilizer such as "Thermo Scientific Protein Stabilizing Cocktail" (Life Technologies, product no. 89806). Any other aqueous solvent or aqueous medium (e.g., containing at least about 60 vol-% water, preferably at least about 70 vol-% water, more preferably at least about 80 vol-% water, even more preferably at least about 90 vol-% water, still more preferably at least about 95 vol-% water) can also be used in place of the above-described aqueous solution in order to ensure the stability of the protein.

In a preferred embodiment, step a) is conducted by providing the luminescent protein in an aqueous solution and subsequently adding the star-like polymer to the aqueous solution of the protein, mixing the protein and the star-like in the aqueous solution, and optionally adding further aqueous solution during said mixing.

It is preferred that said mixing of the luminescent protein and the star-like polymer in step (a) is conducted under stirring (e.g., at 1500 rpm). It is furthermore preferred that step (a) of the processes 1 and 2 is conducted under ambient conditions, particularly at a temperature of about 15°C to about 35°C, preferably at about 20°C to about 30°C, and more preferably at about 25°C.

In another preferred embodiment, the star-like polymer can be any of those mentioned in the description hereinabove.

In step (b) of processes 1 and 2 of the invention a solution of a hydrophobic thermoplastic polymer having a Tg higher than 30°C in a solvent which is orthogonal to water is added to the resulting mixture in the aqueous solution obtained in step (a).

In the context of the present invention, an "orthogonal solvent" refers to a solvent which has at least one opposite property to water in terms of polarity and protons present in the chemical structure thereof, i.e. a solvent which is aprotic or non-polar. Thus, orthogonal solvents may be selected from aprotic and polar solvents, protic and non-polar solvents, and aprotic and non-polar solvents. This solvent should also be such that allows the dissolution of the hydrophobic thermoplastic polymer.

In a preferred embodiment, the orthogonal solvent is selected from butyl acetate, propyl acetate, ethyl acetate,

propylene glycol methyl ether acetate, dioxane, dichloromethane, benzene, toluene, tetrahydrofuran, and acetonitrile. More preferably, the orthogonal solvent is acetonitrile.

The hydrophobic thermoplastic polymer can be any of those mentioned in the description hereinabove.

In a preferred embodiment, step (b) is conducted by providing the hydrophobic thermoplastic polymer in a solvent which is orthogonal to water and subsequently adding said solution to the resulting mixture in the aqueous solution of step (a), thus leading to a mixture of the luminescent protein, the star-like polymer and the hydrophobic thermoplastic polymer in the aqueous solution and the orthogonal solvent, and optionally adding further orthogonal solvent during said mixing.

In this preferred embodiment, the addition of the solution of the hydrophobic thermoplastic polymer to the mixture obtained in step (a) is carried out using slow diffusion technique. This allows the hydrophobic thermoplastic polymer to penetrate into the star-like polymer or to be absorbed in its polymeric matrix. Subsequently, further orthogonal solvent is added and the resulting mixture is subjected to slow stirring to finally leading to a gel-like material.

In a particular embodiment, the weight ratio between the star-like polymer added to the aqueous solution is step (a) and the hydrophobic thermoplastic polymer dissolved in the orthogonal solvent in step (b) ranges from 1:3 to 3:1, more preferably from 1:2 to 2:1, even more preferably is 1:1.

In any case, a skilled in the art would know which weight proportion of polymers can be used provided that the resulting material composition is rigid at ambient temperature.

The processes 1 and 2 of the invention surprisingly allow using non-aqueous solvents while maintaining the stability of the luminescent protein. Thus, contrary to other processes of the prior art, it is possible to use a combination of hydrophilic and hydrophobic polymers without affecting the viability of luminescent proteins, even in the presence of solvents which supposedly could affect the feasibility of said proteins.

Furthermore, the processes 1 and 2 according to the present invention allow the preparation of a material composition containing a protein immobilized therein (or a corresponding gel which can be dried to obtain the rigid material material) without the need for any crosslinking or curing of the polymers that are used. It is thus preferred that the processes 1 and 2 of the invention do not comprise any step of thermally curing, UV curing or crosslinking the polymers that constitute the material composition. It is likewise preferred that the processes 1 and 2 do not comprise any step of covalently crosslinking the polymers that constitute the material composition. Furthermore, in accordance with each one of the various aspects of the present invention, it is preferred that the star-like polymer and the hydrophobic thermoplastic polymer are not covalently crosslinked. It is also preferred that the star-like polymer and the hydrophobic thermoplastic polymer are free of covalent cross-linkages.

Step (c) of process 1 of the invention comprises drying the gel obtained in step (b) in order to obtain the rigid material composition containing the luminescent protein immobilized therein.

In the process 1 of the invention, the combination of hydrophilic and hydrophobic thermoplastics having a Tg higher than 30°C and the subsequent drying of the gel previously formed has led to a rigid matrix which has shown to reduce the temperature of the Bio-LED where it is incorporated under working conditions, thus reducing the loss of water and degradability of proteins.

Preferably, in step (c) the gel is partially dehydrated to obtain the rigid material composition containing the luminescent protein immobilized therein. For example, the gel may be partially dehydrated via vacuum drying, freeze-drying, drum-drying, spray drying, or sunlight-ambient evaporation. It is particularly preferred that the gel is partially dehydrated via vacuum drying.

Accordingly, it is preferred that in step (c) the gel is partially dehydrated in a vacuum station/chamber (e.g., at a pressure of about 1 mbar to about 10 mbar for a period of less than or equal to about 1 hour, or alternatively at a pressure of about $10^{-5}$ bar to about $10^{-9}$ bar for a period of about 5 seconds to about 5 minutes).

Before the gel is introduced into the vacuum station/chamber, it can be deposited onto a substrate using any coating or printing method, particularly a solvent-based technique. Exemplary techniques for depositing the gel onto a substrate include, in particular, doctor-blading, roll-to-roll coating, spin coating, gravure printing, inkjet printing, flexographic printing, screen printing, or 3D printing.

The rigid material composition can be prepared in the form of a film having a thickness of about 10 nm to about 20 mm, preferably of about 10 $\mu$m to 10 mm.

The rigid material composition according to the first and third aspects of the invention can further be deposited onto a substrate/support. Alternatively, the gel according to the fourth aspect of the invention can be dried on a substrate (e.g., as described above in connection with step (c) of the process 1 of the invention) in order to directly obtain the rigid material deposited on the corresponding substrate.

As a further alternative, the rigid material composition deposited on a substrate can also be prepared using a process comprising: (a) introducing a substrate into the gel according to the fourth aspect of the invention (or into the gel obtained in the process 2 according to the invention); and (b) drying the gel on the substrate to obtain a rigid material deposited on the substrate. This latter process is particularly suitable for depositing the rigid material onto a three-dimensional substrate.

The substrate/support onto which the rigid material can be deposited is not particularly limited, and may be selected from organic materials such as carboxymethyl-cellulose, starch, collagen, modified sepharose, ion exchange resins, active charcoal, polymers, active membranes, etc. or from inorganic materials such as silica, clay, metal oxides, diatomaceous earth, hydroxyapatite, ceramic, glass, etc.

The invention also relates to the use of the rigid material composition of the invention containing a luminescent protein immobilized therein as a colour down-converting encapsulation. In accordance with this aspect, the invention also refers to the use of the rigid material composition of the invention as a colour down-converting cascade energy transfer encapsulation for a hybrid LED. Such a cascade energy transfer encapsulation typically comprises or consists of several layers (e.g., two, three, four, five, six, seven, eight, or more layers) of the rigid material containing a luminescent protein immobilized therein, wherein the luminescent proteins in each pair of neighbouring layers have complementary absorption and emission features. The bottom layers may, e.g., emit high energy photons upon excitation from the inorganic LED that are partially converted by the top layer into low-energy photons. The combination of the non-absorbed high-energy photons from the inorganic LED, the bottom down-converting layer, and the top down-converting layer preferably results in a white LED. The rigid material containing a luminescent protein immobilized therein can thus be used for fabricating white light-emitting diodes using an innovative cascade energy transfer encapsulating system, which circumvents the problems related to phase separation and exciton-loss in multicomponent single-layer down-converting encapsulation systems. The encapsulation system features a higher rendering colour with advantageous stabilities under high luminance inputs and advantageous luminous efficiencies when the device is running under ambient conditions.

Furthermore, the light output of the device can be easily modified by the thickness of the rigid encapsulation material, thus covering the whole visible spectrum.

Thus, another aspect of the invention relates to a hybrid light-emitting diode (hybrid LED) which comprises a bottom light emitting inorganic chip or diode (LED), optionally a first encapsulation on the inorganic LED, and a second encapsulation composed of the rigid material composition comprising a luminescent protein immobilized therein. This second encapsulation is preferably multilayered, and may be placed directly on top of the inorganic LED or on top of the first encapsulation (which may be made of an organic and/or an inorganic material, and may have any 3D form). Upon excitation by the inorganic LED, such luminescent proteins partially convert the high-energy photons into low-energy photons, which sum up to the non-absorbed high-energy photons from the inorganic LED, resulting in a colour change of the inorganic LED.

Figure 1 shows a hybrid light-emitting diode according to the present invention, which comprises a substrate (1) with electrical connections, in particular a leadframe (2) to be connected to an electronic driver, a heat sink (3), a light emitting inorganic chip (4) which can be blue- or UV-emitting chip, and a down-converting encapsulation system (5) that consists of one or several layers of the rigid material composition containing the luminescent proteins (6) immobilized therein.

## Examples

Example 1. Preparation of fluorescent proteins.

The fluorescent proteins were prepared following a process consisting of two steps: Cloning of recombinant gene constructs and preparation of fluorescent proteins.

*Cloning of recombinant gene constructs*

To combine the different protein domains and to create the pQE-9 expression constructs, the overlap-PCR method was performed. Using gene specific oligonucleotides eGFP was fused to the SH2-domain (eGFP), mCherry was fused to the SH3-domain (mCherry) and mTagBFP was fused to the PABC-domain (mTagBFP). Fluorescent proteins and protein interaction domains were separated by glycine-serine linker sequences allowing proper folding of both protein domains. The extension of the proteins results in larger and more stable fusion proteins. After PCR and gel extraction the DNA fragments were ligated into the pQE-9 E. coli expression vector that contains an N-terminal 6xHis affinity tag, using T4 DNA ligase. The right orientation of the constructs and the N-terminal in frame fusion with the 6xHis tag were guaranteed using specific restriction enzymes. After the ligation, the recombinant plasmids were transformed into XL1 Blue E. coli cells and the correct sequence of the constructs was verified using Sanger Sequencing (GATC). For expression of the recombinant proteins pQE-9 plasmids, harboring the respective gene constructs, were transformed into E. coli M15 cells carrying the pREP4 repressor plasmid. Transformed E. coli cells were selected on plates containing ampicillin (pQE-9 expression vector, 200 μg/ml) and kanamycin (pREP4 repressor plasmid, 25 μg/ml).

*Preparation of fluorescent proteins*

E. coli strain M15 [pREP4] harboring the appropriate plasmids (pQE-9 expression constructs all containing a N-terminal 6xHis-tag coming from the pQE-9 expression vector, Qiagen) were grown at 28°C in Lysogeny Broth (LB) medium (Bertani G, J Bacteriol. 1951, 62(3), 293-300) containing ampicillin (200 μg/ml) and kanamycin (25 μg/ml) antibiotics to an optical density of approximately 0.5 at 600 nm. Recombinant protein expression was induced with 1 mM isopropyl β-D-1-thiogalactopyranoside at 28°C. After 4 h of induction at 28°C, cells were harvested and frozen at -20°C. Frozen bacteria cells were thawed and lysed by lysozyme treatment and sonication. Recombinant proteins were affinity purified by Ni-NTA affinity chromatography under native conditions, according to instructions of the manufacturer (QIAGEN). The concentration of the resulting purified proteins was determined by measuring the absorption at 280 nm using a NanoDrop Spectrophotometer ND-1000 (Peqlab). The purified proteins were dissolved/stored in elution buffer (50 mM NaH2PO4, pH 8.0; 300 mM NaCl; 250 mM imidazole) until further use.

Example 2. Preparation of a rigid color down-converting encapsulation system onto a LED chip

The first step consisted in the preparation of the protein-based gel-like material according to the present invention. Said protein-based gel was prepared by mixing 60 mg of trimethylolpropane ethoxylate (TMPE) having a Mn of 450 Da with 10-30 μL of an aqueous solution of the fluorescent protein (0.15 mg) obtained according to example 1. Subsequently, 60 mg of poly(methyl methacrylate) (PMMA) having a Mn of 150.000 Da were added to the TMPE/protein mixture and then 100 μL of acetonitrile were also added. The resulting mixture was stirred for 5 minutes at 600 rpm followed by a second addition of 100 μL of acetonitrile while stirring at 600 rpm for 5 minutes more. The mixture was left at room temperature overnight thus forming a gel-like material.

Once the protein-based gel-like material was obtained, this was deposited onto a light-emitting chip wetting completely its surface. In particular, a commercial blue emitting LED chip (purchased by Luxeon) with an electroluminescent spectrum at 450 nm was used in this example.

The resulting coated LED was transferred to a vacuum chamber under 1 mbar for less than 1 hour. This drying step led to the formation of a rigid encapsulation coating onto the LED chip. This procedure was repeated to enhance the light down-conversion efficiency of the Bio-LED with respect to the thickness of the rigid coating which was 1-2 mm.

Comparative example. Preparation of a rubber-like color down-converting encapsulation system onto a LED chip

For comparative purposes, a color down-converting encapsulation system was prepared according to that described in WO2016/203028. Thus, a protein-based gel was also prepared by mixing 120 mg of trimethylolpropane ethoxylate (TMPE) having a Mn of 450 Da with 20 mg of linear poly(ethylene oxide) having Mn of $5x10^6$ Da, and adding to the resulting mixture 200 μL of an aqueous solution containing 0.15 mg of the fluorescent protein as obtained in example 1 while stirring vigorously at 1500 rpm overnight. The mixture was left at room temperature thus forming a gel-like material.

Once the protein-based gel-like material was obtained, this was deposited onto a light-emitting chip wetting completely its surface. In particular, a commercial blue emitting LED chip (purchased by Luxeon) with an electroluminescent spectrum at 450 nm was used in this example.

The resulting coated LED was transferred to a vacuum chamber under 1 mbar for less than 1 hour. This drying step led to the formation of a rubber-like encapsulation coating onto the LED chip. This procedure was repeated to enhance the light down-conversion efficiency of the Bio-LED with respect to the thickness of the coating which was 1-2 mm.

Exemple 3. Characterization of the Bio-LEDs

The Bio-LEDs prepared according to example 2 and comparative example were characterized by using a Keithley 2400 as a current source applying 200 mA, while the luminous efficiency and changes of the electroluminescence spectrum were monitored by using Avantes spectrophotometer (Avaspec-ULS2048L-USB2) in conjunction with a sphere Avasphere 30-Irrad.

Figures 2 and 3 show the device performance of a Bio-LED with a rubber-like color down-converting encapsulation system and a rigid color down-converting encapsulation system, respectively. More particularly, said figures plot the emission intensity of the fluorescent protein and the temperature of the coating upon driving at currents of 200 mA.

As can be observed, the Bio-LED containing the rubber-like color down-converting encapsulation system reaches a higher temperature (close to 45°C). This negatively affects the viability of the fluorescent proteins, leading to a decrease in tis luminescence. In fact, the decrease of temperature over time is the result of the loss of luminescence.

On the contrary, when a rigid color down-converting encapsulation system is used according to the invention, a lower temperature (less than 30°C) is observed under the device working conditions which is maintained over time.

In fact, the loss of luminescence is significantly lower when compared to the Bio-LED having the rubber-like coating.

These results point out that the presence of a hydrophobic thermoplastic polymer having a Tg higher than 30°C provides a high thermal stability to the color down-converting encapsulation system as no temperature increase is observed, thus reducing significantly the degradability of fluorescent proteins and making Bio-LEDs surprisingly more stable over time.

## Claims

1. A rigid material composition containing a luminescent protein immobilized therein, wherein the rigid material composition further comprises a hydrophilic star-like polymer and a hydrophobic thermoplastic polymer, said hydrophobic thermoplastic polymer having a Tg higher than 30°C, and wherein the hydrophilic star-like polymer comprises at least three polymeric branches bound to a central core.

2. The rigid material composition according to claim 1, wherein the Tg of the hydrophobic thermoplastic polymer is higher than 70°C.

3. The rigid material composition according to claims 1 or 2, wherein the hydrophobic thermoplastic polymer is selected from poly(meth)acrylates, vinyl halide polymers, polystyrenes, polycarbonates and mixtures thereof.

4. The rigid material composition according to any of the preceding claims, wherein the hydrophobic thermoplastic polymer is poly(methyl methacrylate).

5. The rigid material composition according to any of the preceding claims, wherein the star-like polymer is selected from trimethylolpropane ethoxylate, trimethylolpropane ethoxylate methyl ether diacrylate and polyethyleneimine.

6. The rigid material composition according to any of the preceding claims, wherein the star-like polymer has a number average molecular weight of 200 Da to 2000 Da.

7. The rigid material composition according to any of the preceding claims, wherein the hydrophobic thermoplastic polymer is poly(methyl methacrylate) and the star-like polymer is trimethylolpropane ethoxylate.

8. The rigid material composition according to any of the preceding claims, wherein the weight ratio of the hydrophobic thermoplastic polymer to star-like polymer ranges from 1:3 to 3:1.

9. The rigid material composition according to any of the preceding claims, wherein the luminescent protein in a fluorescent protein.

10. A process for producing a rigid material composition as defined in claims 1 to 9, said process comprising:

    a) mixing the luminescent protein and the star-like polymer in an aqueous solution;
    b) adding to the mixture resulting from step a) a solution of a hydrophobic thermoplastic polymer having a Tg higher than 30°C in an orthogonal solvent to water, to obtain a gel-like material; and
    c) drying the gel obtained in step b) to obtain a rigid material composition containing the luminescent protein immobilized therein.

11. The process according to claim 10, wherein the orthogonal solvent is selected from butyl acetate, propyl acetate, ethyl acetate, propylene glycol methyl ether acetate, dioxane, benzene and acetonitrile.

12. A rigid material composition obtainable by the process as defined in claims 10 to 11.

13. A gel-like material comprising a luminescent protein, a hydrophilic star-like polymer and a hydrophobic thermoplastic polymer, said hydrophobic thermoplastic polymer having a Tg higher than 30°C, and wherein the hydrophilic star-like polymer comprises at least three polymeric branches bound to a central branching unit.

14. Use of the rigid material composition as defined in claims 1 to 9 and 12, as an environmentally friendly color down-converting encapsulation material for a hybrid light-emitting diode.

**15.** A hybrid light emitting-diode comprising a light-emitting diode and a color down-converting encapsulation material, said color down-converting encapsulation material comprise at least one layer of rigid material composition containing a luminescent protein immobilized therein as defined in any of claims 1 to 9 and 12.

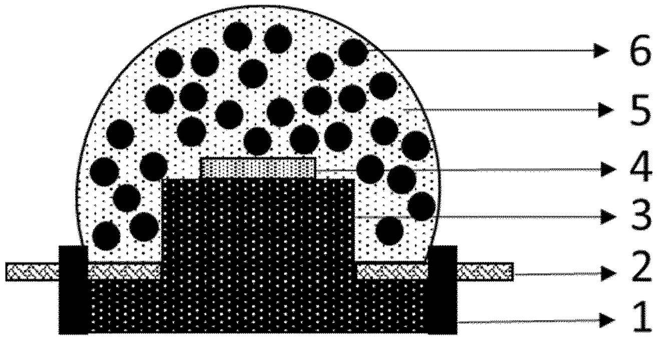


Figure 1

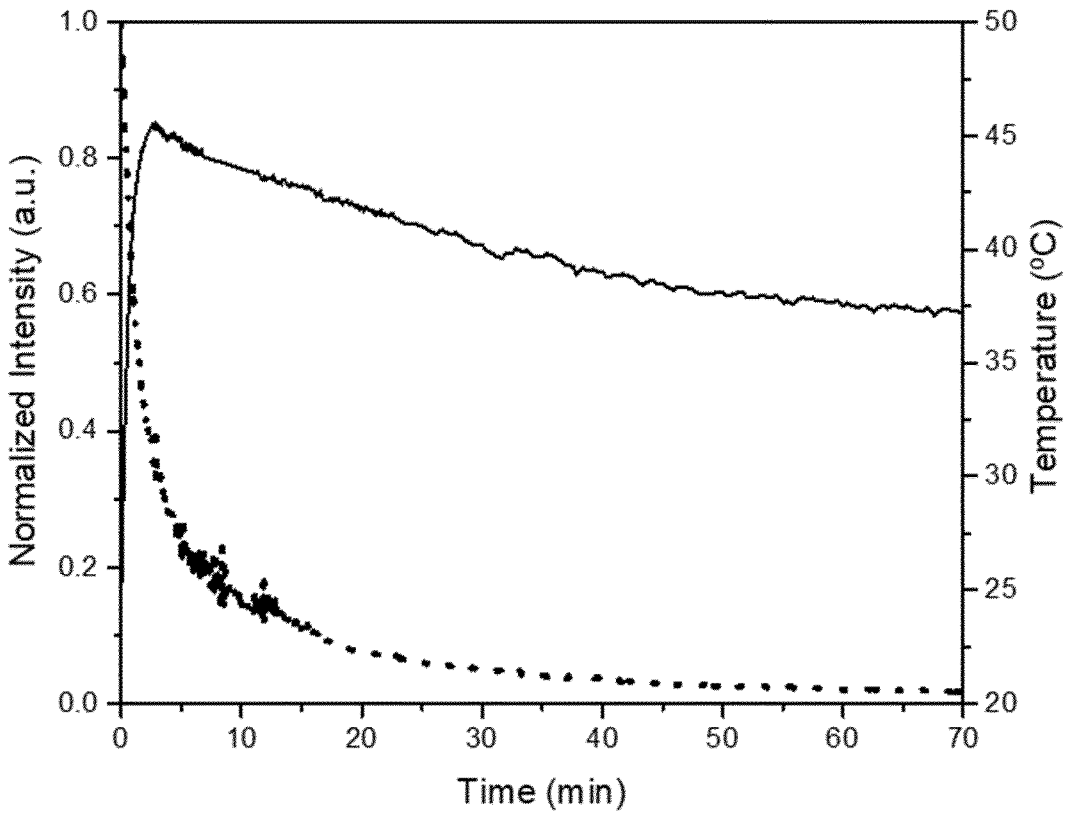


Figure 2

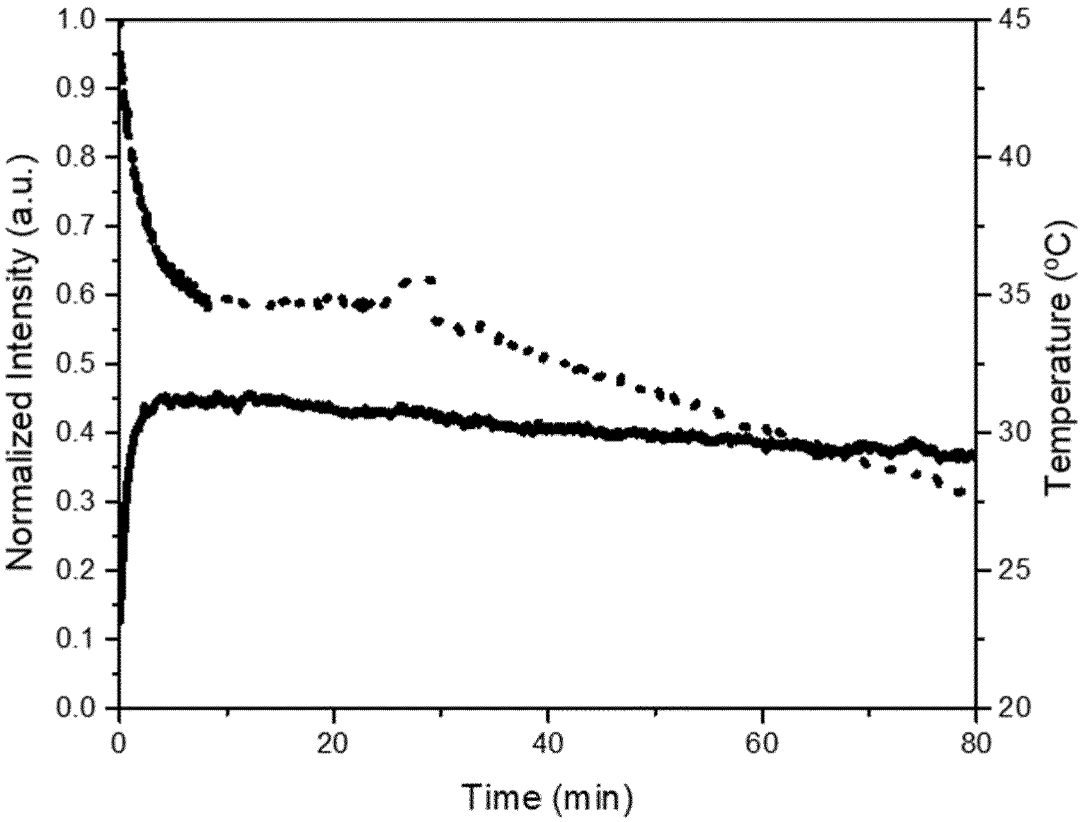
1.0
0.9
0.8
0.7
0.6
0.5
0.4
0.3
0.2
0.1
0.0
Normalized Intensity (a.u.)
45
40
35
30
25
20
Temperature (ºC)
0
20
40
60
80
Time (min)

Figure 3

Europäisches Patentamt
European Patent Office
Office européen des brevets

# EUROPEAN SEARCH REPORT

Application Number
EP 17 38 2840

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | WO 2016/203028 A1 (FRIEDRICH-ALEXANDER-UNIVERSITÄT ERLANGEN-NÜRNBERG [DE]) 22 December 2016 (2016-12-22) * the whole document * ----- | 1-15 | INV. C07K17/04 C09K11/06 H05B33/14 |
| A,D | DANIEL AARON PRESS ET AL: "Fluorescent protein integrated white LEDs for displays", NANOTECHNOLOGY, IOP, BRISTOL, GB, vol. 27, no. 45, 7 October 2016 (2016-10-07), XP020310360, ISSN: 0957-4484, DOI: 10.1088/0957-4484/27/45/45LT01 [retrieved on 2016-10-07] * the whole document * ----- | 1-15 | |
| X | TOBIAS BOLLHORST ET AL: "Colloidal capsules: nano- and microcapsules with colloidal particle shells", CHEMICAL SOCIETY REVIEWS, vol. 46, no. 8, 1 January 2017 (2017-01-01), pages 2091-2126, XP055460435, ISSN: 0306-0012, DOI: 10.1039/C6CS00632A * the whole document * * page 2096 - page 2097 * * page 2110 - page 2111 * ----- | 1-13 | TECHNICAL FIELDS SEARCHED (IPC) C07K C12N |
| | -/-- | | |

The present search report has been drawn up for all claims

1

| Place of search | Date of completion of the search | Examiner |
|---|---|---|
| Munich | 20 March 2018 | Cervigni, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 17 38 2840

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ANDREW GREGORY ET AL: "Complex polymer architectures via RAFT polymerization: From fundamental process to extending the scope using click chemistry and nature's building blocks", PROGRESS IN POLYMER SCIENCE, PERGAMON PRESS, OXFORD, GB, vol. 37, no. 1, 17 August 2011 (2011-08-17), pages 38-105, XP028334451, ISSN: 0079-6700, DOI: 10.1016/J.PROGPOLYMSCI.2011.08.004 [retrieved on 2011-08-25] * the whole document * * page 392 * ----- | 1-13 | |
| X | GREINER A ET AL: "Biohybrid nanosystems with polymer nanofibers and nanotubes", APPLIED MICROBIOLOGY AND BIOTECHNOLOGY, SPRINGER, BERLIN, DE, vol. 71, no. 4, 10 June 2006 (2006-06-10), pages 387-393, XP019422044, ISSN: 1432-0614, DOI: 10.1007/S00253-006-0356-Z * the whole document * ----- | 1-13 | TECHNICAL FIELDS SEARCHED (IPC) |
| X | MUHAMMET U. KAHVECI ET AL: "Photoinduced in situ formation of clickable PEG hydrogels and their antibody conjugation", DESIGNED MONOMERS AND POLYMERS, vol. 18, no. 2, 27 October 2014 (2014-10-27), pages 129-136, XP055460522, DOI: 10.1080/15685551.2014.971392 * the whole document * ----- -/-- | 1-13 | |

The present search report has been drawn up for all claims

1

| Place of search | Date of completion of the search | Examiner |
|---|---|---|
| Munich | 20 March 2018 | Cervigni, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 17 38 2840

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | MARCOS RODRIGUES FACCHINI CERQUEIRA ET AL: "Use of poly(methyl methacrylate)/polyethyleneimine flow microreactors for enzyme immobilization", MICROCHEMICAL JOURNAL, vol. 118, 1 January 2015 (2015-01-01), pages 231-237, XP055460524, US ISSN: 0026-265X, DOI: 10.1016/j.microc.2014.09.009 * the whole document * ----- | 1-13 | |

TECHNICAL FIELDS SEARCHED (IPC)

The present search report has been drawn up for all claims

1

| Place of search | Date of completion of the search | Examiner |
|---|---|---|
| Munich | 20 March 2018 | Cervigni, S |

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 3 of 3

## ANNEX TO THE EUROPEAN SEARCH REPORT ON EUROPEAN PATENT APPLICATION NO. EP 17 38 2840

This annex lists the patent family members relating to the patent documents cited in the above-mentioned European search report.
The members are as contained in the European Patent Office EDP file on
The European Patent Office is in no way liable for these particulars which are merely given for the purpose of information.

20-03-2018

| Patent document cited in search report | Publication date | Patent family member(s) | Publication date |
|---|---|---|---|
| WO 2016203028 A1 | 22-12-2016 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2017039406 A
- WO 2016203028 A


### Non-patent literature cited in the description


- **PARK JK et al.** *Appl Phys Lett.,* 2004, vol. 84, 1647
- **JANG HS et al.** *Appl Phys Lett.,* 2007, vol. 90, 041906
- **XIE R-J et al.** Nitride Phosphors and Solid-State Lighting. CRC Press, 2011
- **TOLHURST TM et al.** *Adv Opt Mater.,* 2015, vol. 3, 546
- **ZHANG R et al.** *Laser Photon Rev.,* 2014, vol. 8, 158
- *J. Am. Ceram. Soc.,* 2014, vol. 97, 1327-1352
- *J. Sol. State Light,* 2014, vol. 1, 11
- *Environ. Health Perspect.,* 2014, vol. 122, 269
- *Curr. Pharm. Des.,* 2015, vol. 21, 3428
- *Neuroscience,* 2016, vol. 339, 296
- Innovative Materials: Engineering and Applications II. Key Engineering Materials. Trans Tech Publications, 2017, vol. 730, 112
- *Lighting Research & Technology,* 2017, https://doi.org/10.1177/1477153517708597
- **HELIOTIS G et al.** *Adv Mater.,* 2006, vol. 18, 334
- **GU E. et al.** *Appl Phys Lett.,* 2007, vol. 90, 031116
- **HUYAL IO et al.** *J Mater Chem.,* 2008, vol. 18, 3568
- **KIM O et al.** *ACS Nano,* 2010, vol. 4, 3397
- **STUPCA M et al.** *J Appl Phys.,* 2012, vol. 112, 074313
- **BAN D et al.** *Phys Status Solidi Curr Top Solid State Phys.,* 2012, vol. 9, 2594
- **JANG E-P et al.** *Nanotechnology,* 2013, vol. 24, 045607
- **FINDLAY NJ et al.** *J Mater Chem C,* 2013, vol. 1, 2249
- **LAI C-F et al.** *Opt Lett.,* 2013, vol. 38, 4082
- **CHEN J et al.** *J Mater Sci.,* 2014, vol. 49, 7391
- **KIM J-Y.** *Opt Commun.,* 2014, vol. 321, 86
- **SHEN P-C et al.** *Sci Rep.,* 2014, vol. 4, 5307
- **FINDLAY NJ et al.** *Adv Mater.,* 2014, vol. 26, 7290
- *Adv. Mater.,* 2006, vol. 18, 334
- *J. Mater. Chem.,* 2008, vol. 18, 3568
- *ACS Nano,* 2010, vol. 4, 3397
- *Nanotechnology,* 2013, vol. 24, 045607
- *Sci. Rep.,* 2014, vol. 4, 5307
- *Adv. Mater.,* 2014, vol. 26, 7290
- *Nanoscale,* 2015, vol. 7, 12045
- *Nat. Commun.,* 2013, vol. 4, 2717
- *Adv. Funct. Mater.,* 2015, vol. 25, 4796
- *J. Mater. Chem. C,* 2015, vol. 3, 3536
- *ACS Appl. Mater. Interfaces,* 2015, vol. 7, 15830
- *Mater. Horiz.,* 2016, vol. 3, 340
- *Adv. Mater.,* 2015, vol. 27, 5493
- *Adv. Funct. Mater.,* 2017, vol. 27, 1601792
- **HUI, K.N.** *Nanotechnology,* 2008, vol. 19, 355203
- *Fen Bilim. Enstitüsü Derg.,* 2016, vol. 20, 490
- *Nanotechnology,* 2016, vol. 27, 45LT01
- *Nanotechnology,* 2008, vol. 19, 355203
- **REDDY CHICHILI VP et al.** *Protein Scí.,* 2013, vol. 22 (2), 153-67
- **BERTANI G.** *J Bacteriol.,* 1951, vol. 62 (3), 293-300